# EUROPEAN PATENT SPECIFICATION

(11) **EP 2 982 763 B1**
(45) Date of publication and mention of the grant of the patent: **07.03.2018**
(21) Application number: 14002761.6
(22) Date of filing: 07.08.2014
(51) Int. Cl.: C12Q 1/68, C12Q 1/70

(54) **Synthesis and use of a polyprobe for the detection of viral infections in globe artichoke**
Synthese und Verwendung einer Mehrfachsonde zum Nachweis viraler Infektionen in Artischocken
Synthèse et utilisation d'un polyprobe pour la détection d'infections virales dans un artichaut

(43) Date of publication of application: 10.02.2016
(73) Proprietor: Agritest S.r.l., 70010 Valenzano (BA) (IT)
(72) Inventor: Minutillo, Serena Anna, I-70126 Bari (IT); Mascia, Tiziana, I-70126 Bari (IT); Gallitelli, Donato, I-70126 Bari (IT)
(74) Representative: Trupiano, Federica

(56) References cited:
- EP-A2- 1 577 399
- WO-A2-2005/030929
- SERENA ANNA MINUTILLO ET AL: "A DNA probe mix for the multiplex detection of ten artichoke viruses", EUROPEAN JOURNAL OF PLANT PATHOLOGY, KLUWER ACADEMIC PUBLISHERS, DO, vol. 134, no. 3, 14 September 2012 (2012-09-14), pages 459-465, XP035117879, ISSN: 1573-8469, DOI: 10.1007/S10658-012-0032-3
- FREDERIC APARICIO ET AL: "Simultaneous detection of six RNA plant viruses affecting tomato crops using a single digoxigenin-labelled polyprobe", EUROPEAN JOURNAL OF PLANT PATHOLOGY, KLUWER ACADEMIC PUBLISHERS, DO, vol. 123, no. 1, 8 July 2008 (2008-07-08), pages 117-123, XP019641560, ISSN: 1573-8469
- GALLITELLI DONATO ET AL: "Viruses in artichoke.", ADVANCES IN VIRUS RESEARCH 2012, vol. 84, 2012, pages 289-324, XP8174680, ISSN: 1557-8399

## Description

The present invention concerns a process for the synthesis of a molecular polyprobe comprising nucleic acid useful for simultaneous diagnosis of several harmful viruses affecting the quality of artichoke production, in particular in nursery productions, a diagnostic kit comprising said molecular polyprobe and its use for detecting artichoke viruses.

### PRIOR ART

Globe artichoke represents an important part of the human diet, and the market appreciation that is today granted to this plant depends not only on its organoleptic characteristics but, in increasing measure, also on pharmaceutical and biomedical issues. Globe artichoke turns out to be particularly rich of compounds that protect biological molecules (proteins, lipids and DNA) from oxidative stresses caused from free radicals, and also have hepatoprotective and choleretic activities. Such therapeutic properties depend on the presence of secondary compounds deriving from the phenylpropanoid metabolism (luteoline, chlorogenic acid, cynarine and caffeic acid), that show a sharp anti-oxidant activity and could also contribute to the prevention of arteriosclerosis and of vascular diseases. Artichoke leaf extracts, thanks to the cited secondary metabolites, have been shown able to protect human leucocytes and rat hepatocytes from oxidative stresses in a dose-dependant manner. The presence of viruses in globe artichoke, as well as in other vegetable crops, diminish its nutritional and nutraceutical properties while reducing crop yield and quality. Traditional artichoke types are propagated vegetatively from at most visually inspected mother plants, which has led overtime several pathogens to accumulate, such as vascular fungi (mainly Verticillium spp.) and viruses. This has led to a progressive degeneration of the crop, which reflects on the poor quantity and quality of produce.

Plant viruses are difficult to control because of their intimate dependence from intracellular transcription and expression machinery. One way for control is through their natural vectors - insects, mites, nematodes, and fungi - by the recourse to heavy pesticide treatments, which is opposing to the integrated pest management (IPM) strategies of the EU. Concerns about the risks posed by the current use of pesticides have led the EU to adopt a new legislative "pesticides package"; a regulatory context that force farmers to face the future challenge of protecting their crops with an IPM strategy. These considerations together the damaging effects of virus infections on the quantity and quality of the yield highlight that new artichoke fields must be planted with virus-free and with a certified phytosanitary status propagation material. Its availability would reduce the problem and allow a great increase in productivity. In addition, it would make nursery productions conform to the current EU legislation, which needs that nursery productions originate from virus-free and true-to-type mother plants. Seed propagation has been regarded as an attractive means to make available virus-free plants. However, most artichoke-infecting viruses are seed and pollen-transmitted. Thus, procedures to detect also seed-transmitted viruses must be applied if seed-based nursery productions will be grown.

At the present, no treatments are available to heal an artichoke, once it has been infected by a virus.

Taking the above into account, prevention measures must be adopted, for the early diagnosis of plant virus infections on asymptomatic vegetal material, as in the typical case of nursery productions.

The diagnostic instruments available today, essentially consist of tests on biological indicators, serological tests and the so-called tests based on the properties of nucleic acid.

Tests on biological indicators usually request long times and high costs, while serological tests, which are very practical, are not always reliable, due to poor immunogenicity of some viruses, and are not suitable for detecting viroids because they have no protein envelope.

Assays based on the properties of nucleic acid (hereafter denoted molecular) overcome most of the limits of other tests even guaranteeing high sensibility and versatility in use. Modern molecular tests allow the detection of specific viral sequences even in raw extracts of plant tissues and do not require the use of radioactive markers, allowing them to be proposed also in commercial kits that are easy to use. For example, presently available commercial kits, comprise one or more DNA single specific probes that could hybridize with single specific viral target sequences, leading to the detection of the infection and to the identification of the specific type of virus. These kits are based on single probes that can be used singly or in mixture. The manufacture of this kind of kits, requests a great quantity of reagents, that means high costs and long times of production. Furthermore, unless specifically required by phytosanitary extension services, the specific identification of all the single virus species in a plant sample is an unnecessary information for the certification purposes, as it is sufficient to verify that the it does not contain any of the viruses listed in the phytosanitary certificate. In this view, costs for the production of single specific probes for the identification of specific single viruses are therefore unnecessary.

An alternative to the mixture of single probes is the use of polyprobes. A polyprobe consists of a series of sequences specific for a single virus species joined together in a single fragment. This strategy allows the simultaneous detection of different viruses depending on the number of specific sequence present in the polyprobe. However, the known processes for the synthesis of polyprobes are complex and elaborate, and require a large quantity of materials, reagents and time. At the state of the art, to carry out the synthesis of polyprobes by means of known processes, infected plant material is needed in order to obtain the viral nucleic acid sequence of interest. In particular, total nucleic acids are extracted from a sample of infected plant, and viral target sequences are identified by means of PCR using specifically designed primers. The products of the amplification are isolated and individually cloned into suitable vectors. The single sequences are then subcloned in tandem in order to obtain a single polyprobe. To carry out this complex process, long preliminary operations are needed. First of all, is fundamental to have available plant samples infected by the specific virus of interest; once the infected plant material is available, is necessary to extract the totality of the nucleic acids and then isolate and clone the viral sequence of interest. Furthermore, most of the known polyprobes are constructed by cloning in tandem selected fragments of viral sequences of variable length. Variability in the length of the selected fragment is often required to obtain compatible restriction cleavage sites between the two adjacent sequences. In view of this need for compatible restriction cleavage sites, the selection and the analysis of the sequences is one of the problematic aspects of the known processes for synthesis of polyprobes. Furthermore, as stated above, the known processes of synthesis of polyprobes, need preliminary operations requiring, as essential starting point, the availability of plant samples specifically infected by the virus of interest, and involving long working time, a lot of procedural steps and, consequently, higher consumption of reagents and higher costs of realization.

Document "Simultaneous detection of six RNA plant viruses affecting tomato crops using a single digoxigenin-labelled polyprobe", FREDERIC APARICIO ET AL., EUROPEAN JOURNAL OF PLANT PATHOLOGY, KLUWER ACADEMIC PUBLISHERS, DO, vol. 123, no. 1, 8 July 2008 (2008-07 -08), pages 117-123, XP019641560, ISSN: 1573-8469, discloses the use of a polyprobe for the simultaneous detection of 6 viruses in tomato. To construct the polyprobe of the document "Simultaneous detection of six RNA plant viruses affecting tomato crops using a single digoxigenin-labelled polyprobe", total nucleic acids were extracted from fresh leaf tissue of infected tomato plants. Primers were designed to amplify a fragment of around 200 bp length corresponding to a region of the coating protein genes of the viruses. Amplified products corresponding to the six viral CP (coat protein genes) were cloned individually into a vector; in a second step, the cloned fragments were subcloned in tandem into a pSK+ vector using the corresponding restriction enzymes. A vector containing the fusion of all six viral sequences was thus obtained.

Document WO2005/030929A2 discloses a polymeric nucleic acid hybridization probe made up of multiple copies of a nucleic acid probe sequence, which is complementary to a sequence of interest in the target nucleic acid.

Document EP1577399A2 discloses a diagnostic kit for detecting one or more plant viruses and/or viroids and their variants from among *Vitivirus Grapevine virus A, Grapevine virus B, Grapevine virus D, Grapevine leafroll-associated virus 4, Grapevine leafroll-associated virus 5, Grapevine fleck virus, Grapevine red globe virus, Grapevine asteroid mosaic virus, Grapevine leafroll-associated virus. 7, Plum bark neurosis stem pitting-associated virus, Peach latent mosaic viroid, variants of Peach latent mosaic viroid responsible for calico of the peach, Plum pox virus, Citrus psorosis virus, Citrus variegation virus, Citrus tristeza virus, Olive latent virus-1, Olive latent virus,-2, Olive latent ringspot virus, Olive latent yellowing associated virus, Turnip mosaic virus, Alfalfa mosaic virus, Cucumber mosaic virus, Impatiens neurotic spot virus, Tomato spotted wilt virus, Pelargonium zonate spot virus, Lettuce mosaic virus, Cucumber green mottle mosaic virus, Bean yellow mosaic virus, Celery mosaic virus, Artichoke mottled crinkle virus, Artichoke latent virus and Artichoke Italian latent virus,* that comprises PCR primers and/or cloned nucleotide sequences and/or nucleic probes.

In view of the present state of the art, cheaper and faster procedures for the production of polyprobes are needed. Furthermore, there is a need for new polyprobes useful to make the mass analysis of, for example, globe artichoke, easier and faster, in order to simplify and speed up the analysis of nursery vegetable productions and consequently certification procedures.

### SUMMARY OF THE INVENTION

The present invention solves the critical problems of the present state of the art.

A first object of the present invention is to make available a method for the synthesis of polyprobes, which can be prepared according to cheaper and faster procedures.

A second object of the present invention is to make available polyprobes, which allow to simplify and speed up the analysis of nursery globe artichoke productions. A third object of the present invention is to make available polyprobes, which allows to simplify and speed up the required certification procedures for nursery globe artichoke productions.

A fourth object of the present invention is to make available a diagnostic kit for the detection of viral infections in globe artichoke, easy to be used and reliable on given results.

A sixth object of the present invention is to make available a method for the detection of plant viruses in globe artichoke samples, which contemplates the use of a diagnostic kit according to the present invention.

### DETAILED DESCRIPTION OF THE INVENTION

The present invention relates to a method for the synthesis of at least one nucleic acid polyprobe, said polyprobe being based on specifically selected portions, of the genome of plant viruses infecting globe artichoke.

Said specifically selected portions of plant virus genome are genomic target sequences conveniently selected depending on the plant and virus involved.

The innovation of the present invention is that the method, which the present invention relates to, allows the selection and the use of target sequences that are specific, small and, preferably, all of the same length. Sequences with all these features are very difficult to obtain by means of the known methods due to the absence of suitable restriction sites.

The term "polyprobe" refers to a single molecular probe composed of at least two specific genomic target sequences, related to viruses of interest, joined together.

According to the present invention, the wording "genomic target sequence/s" refers to 10 sequences of nucleic acid that are selected from the entire genome of 10 viruses of interest, i.e. a specifically selected portion of the entire genome of 10 viruses of interest.

Preferably, said selected target sequences are specific for each virus species considered and conserved in the known variants/strains/isolates of the same virus species, in order to be able to detect any of the said variants/strains/isolates.

Always according to the present invention, the term "nucleic acid" refers to DNA, RNA and all the naturally and synthetically occurring variants.

The term "plant viruses" refers to any virus that could affect a plant of globe artichoke.

The method of synthesis of a polyprobe according to the present invention, comprises at least the following phases:
a) Selecting 10 genomic target sequences, of 10 globe artichoke's viruses of interest,
b) Synthesizing 4 single-stranded oligonucleotides comprising at least 2 of said genomic target sequences joined together,
c) Synthesizing 4 double-stranded oligonucleotides corresponding to said 4 single-stranded oligonucleotide,
d) cloning said 4double-stranded oligonucleotides into 4 suitable vectors; which are subcloned in tandem obtaining a recombinant cloning vector comprising the entire sequence of said polyprobe,
e) labeling said polyprobe.

According to the present invention, the number of the selected target sequences is 10.

The terms "synthetic oligonucleotides" and "synthetic single-stranded oligonucleotides" refer to single-stranded nucleic acid molecules comprising at least two selected genomic target sequences, of at least two plant viruses of interest, joined together.

Said synthetic single stranded oligonucleotides are synthesized providing, at both of their ends, compatible restriction sites.

The synthesis of said synthetic single-stranded oligonucleotides may be performed by means of techniques known in the art.

The use of said synthetic single-stranded oligonucleotides for the synthesis of a polyprobe, advantageously allows to avoid the long preliminary operations of the known processes, i.e. obtain plant samples that are infected by the specific virus of interest, extract the totality of the nucleic acids from said samples, isolate the viral sequences of interest, synthesize a double-stranded nucleic acid (cDNA) with ends compatible to those of the cloning vector and clone said cDNA. The use of said synthetic single-stranded oligonucleotides makes the process for the synthesis of polyprobes, which the present invention relates to, surprisingly faster, easier and cheaper compared to known processes; furthermore, the use of said synthetic single-stranded oligonucleotides allows to synthesize a highly specific polyprobe selecting short, highly specific, genomic target sequences in order to obtain a polyprobe that combines the best specificity and the shortest length possible. This combination of desirable features cannot be obtained by means of the known processes of synthesis that requires the joining of fragments on the basis of compatible restriction cleavage sites available already on the sequence itself or ligated to it in the form of synthetic oligonucleotides. In particular, in the first case, on the basis of cleavage sites available already on the sequence, it is very difficult to obtain sequences that are at the same time short, specific, and preferably all of the same length; in the latter case, the process of synthesis would be even more complex, and requiring long times and costs of production.

Said synthetic single-stranded oligonucleotides may be used to obtain corresponding double-stranded oligonucleotides which may be individually cloned into suitable vectors, which may be further used to subclone in tandem said double-stranded oligonucleotides in order to obtain a single polyprobe.

Synthetic single stranded oligonucleotides according to the present invention are in number of 4.

A synthetic single stranded oligonucleotide comprises at least 2 selected genomic target sequences joined together.

According to the present invention, a synthetic single stranded oligonucleotide according to the present invention comprises 2 or 3 selected genomic target sequences joined together.

In a preferred embodiment, genomic target sequences according to the present invention are 20 - 2000 nucleotides in their length.

In another preferred embodiment, genomic target sequences according to the present invention are 30 - 500 nucleotides in their length.

In a still another preferred embodiment, genomic target sequences according to the present invention are 40 - 100 nucleotides in their length.

In a more preferred embodiment, genomic target sequences according to the present invention are 45 - 75 nucleotides in their length.

In a most preferred embodiment, genomic target sequences according to the present invention are 50 nucleotides in their length.

In a particularly preferred embodiment, selected genomic target sequences according to the present invention are all of the same length.

According to the invention, a preferred suitable vector is a plasmid.

For example, a plant virus of interest, is a virus that affects an *Embryophyta* plant. Other plant viruses of interest, are viruses that affect the following plants: *Tracheophyta* plant, *Euphyllophyta* plant, *Spermatophyta* plant, *Angiosperm*, *Asteraceae.*

According to the present invention, the plant virus of interest is a virus that affects *Cynara scolymus*, i.e. a virus that may affect artichoke.

In addition to the above, as well as viruses, the present invention also relates to viroids.

Always according to the invention, plant viruses of interest are selected from a group *comprising: Artichoke Italian latent virus* (AILV); *Artichoke latent virus* (ArLV); *Artichoke mottled crinkle virus* (AMCV); *Turnip mosaic virus* (TuMV); *Tomato infectious chlorosis virus* (TICV); *Bean yellow mosaic virus* (BYMV); *Cucumber mosaic virus* (CMV); *Pelargonium zonate spot virus* (PZSV); *Tomato spotted wilt virus* (TSWV), *Tobacco mosaic virus* (TMV), *Artichoke Aegean ringspot virus* (AARSV), *Artichoke yellow ringspot virus* (AYRSV), *Tomato black ring virus* (TBRV), *Artichoke vein banding virus* (AVBV), *Broad bean wilt virus* (BBWV), *Tobacco steak virus* (TSV), *Artichoke latent virus M* (ArLVM), *Artichoke latent virus S* (ArLVS), *Potato virus X* (PVX), *Artichoke curly dwarf virus* (ACDV) and *Tobacco rattle virus* (TRV).

The terms "digestion", "digestion reaction" and "enzymatic digestion" refer to a reaction of cleavage that occurs when a nucleic acid is treated with one or more enzymes, in particular one or more nucleases, i.e. endonucleases and/or esonucleases. Nucleases are enzymes that cleave the phosphodiester bond within a polynucleotide chain. Nucleic acid fragments cleaved by the same nuclease can be joined together regardless of the origin of the nucleic acid. Such nucleic acid is called recombinant. For example, DNA fragments cleaved by the same endonuclease can be joined together regardless of the origin of the DNA. Such DNA is called recombinant DNA. The terms "ligation", "ligation reaction" and "enzymatic ligation" refer to the joining of two nucleic acid fragments by means of the action of an enzyme, i.e. a ligase. The nucleic acid fragments may be derived from a digestion reaction of a longer sequence of nucleic acid. The joining of nucleic acid fragments by means of an enzymatic reaction creates a recombinant nucleic acid molecule. For example, the joining of two DNA fragments by means of an enzymatic reaction creates a recombinant DNA molecule, such as when a foreign DNA fragment is inserted into a suitable vector, for example a plasmid.

In a preferred embodiment the method of synthesis of a polyprobe according to the present invention comprises the following phases:
a) Selecting 10 genomic target sequences of the globe artichoke's viruses of interest, one sequence for each virus,
b) Synthesizing 4 single-stranded oligonucleotides, 2 of them comprising each one 2 of said 10 selected genomic target sequences while the other 2 comprising each one 3 of said 10 selected genomic target sequences,
c) Synthesizing 4 double-stranded oligonucleotides corresponding to said 4 single-stranded oligonucleotides,
d) cloning said 4 double-stranded oligonucleotides, each one into a suitable vector, to obtain 4 recombinant vectors,
e) use said 4 recombinant vectors to synthetize a single recombinant cloning vector comprising all the 10 selected sequences linked together, by means of enzymatic digestion and ligation reactions; the recombinant cloning vector thus obtained comprising the entire sequence of a polyprobe according to the present invention,
f) use said recombinant cloning vector comprising the entire sequence of a polyprobe as template in PCR reactions in order to obtain a labeled polyprobe.

For example, in order to obtain a polyprobe for the simultaneous detection of 10 viruses in artichoke, 4 single-stranded oligonucleotides may have the following sequences:

Is it possible to make available at least one nucleic acid polyprobe, for the simultaneous detection of at least two plant viruses, said polyprobe being based on specific genomic target sequences of plant viruses.

According to the invention, a polyprobe, comprises 10 genomic target sequences of plant viruses of interest, each one from a different virus species.

In a preferred embodiment, genomic target sequences according to the present invention are 20 - 2000 nucleotides in their length.

In another preferred embodiment, genomic target sequences according to the present invention are 30 - 500 nucleotides in their length.

In a still another preferred embodiment, genomic target sequences according to the present invention are 40 - 100 nucleotides in their length.

In a more preferred embodiment, genomic target sequences according to the present invention are 45 - 75 nucleotides in their length.

In a most preferred embodiment, genomic target sequences according to the present invention are 50 nucleotides in their length.

In a particularly preferred embodiment, selected genomic target sequences according to the present invention are all of the same length.

For example, a plant virus of interest, is a virus that affects an *Embryophyta* plant. Other plant viruses of interest, are viruses that affect the following plants: *Tracheophyta* plant, *Euphyllophyta* plant, *Spermatophyta* plant, *Angiosperm, Asteraceae.*

According to the present invention, the plant virus of interest is a virus that affects *Cynara scolymus,* i.e. a virus that may affect artichoke.

A nucleic acid polyprobe is useful for the simultaneous detection of at least two plant viruses in *Embryophyta* plants, *Tracheophyta* plants, *Euphyllophyta* plants, *Spermatophyta* plants, *Angiosperm* plants, *Asteraceae.* According to the invention a nucleic acid polyprobe, is useful for the simultaneous detection of at least two plant viruses in tissue samples of *Cynara scolymus,* i.e. artichoke.

A polyprobe according to the present invention comprises 10 genomic target sequences of plant viruses of interest, each one from a different virus, said viruses being *Artichoke Italian latent virus* (AILV); *Artichoke latent virus* (ArLV); *Artichoke mottled crinkle virus* (AMCV); *Turnip mosaic virus* (TuMV); *Tomato infectious chlorosis virus* (TICV); *Bean yellow mosaic virus* (BYMV); *Cucumber mosaic virus* (CMV); *Pelargonium zonate spot virus* (PZSV); *Tomato spotted wilt virus* (TSWV) e *Tobacco mosaic virus* (TMV).

A nucleic acid polyprobe according to the present invention, is a DNA polyprobe or an RNA polyprobe.

In a more preferred embodiment, a nucleic acid polyprobe according to the present invention, is a DNA polyprobe.

A nucleic acid polyprobe according to the present invention, is useful for the simultaneous detection of at least 2 viruses in artichoke samples.

A polyprobe, according to the present invention, has the following sequence:

The polyprobe, according to the present invention, is non-radioactively labeled.

The polyprobe, according to the present invention, is labeled with a label selected from the group comprising digoxigenin, biotin, fluorescein and tetramethylrhodamine.

For example, the polyprobe, according to the present invention, is labeled with digoxigenin.

Summarizing, the present invention solves the problems of the prior art. In fact, known polyprobes present several problems: for example, known polyprobes only comprise a small number of genomic target sequence, in view of the fact that the necessity of compatible restriction cleavage sites between two adjacent sequences force to use sequences that could be significantly longer with respect to selected target sequences of the present invention, and that usually are, disadvantageously, different in their length. For comparative quantifications is indeed highly desirable that all the probe sequences have the same length. Furthermore, as discussed above, the known processes for the synthesis of known polyprobes need a large amounts of materials, long times, complex and expensive technical operations to obtain each polyprobe.

Therefore, known polyprobes require hard work to be manufactured, thus making known polyprobes particularly disadvantageous when compared with polyprobes according to the present invention. The present invention solves the problems involved with the use of polyprobes according to the prior art, providing a fast, easy to handle and cheaper method for the synthesis of polyprobes. The innovation of the present invention is that the method, which the present invention relates to, allows the selection and the use of target sequences that are specific, small and, preferably, all of the same length. Sequences with all these features that are very difficult to obtain by means of the known methods due to the absence of suitable restriction sites. In particular, step b) of the process according to the invention, allows to use synthetic single-stranded oligonucleotides comprising 10 selected genomic target sequences, as starting material for the synthesis of polyprobes, namely polyprobes for the detection of viral infections in globe artichoke. This process avoids the waste of large amount of materials and waste of time, avoiding complex and expensive operations required by the known processes, but at the same time allows the use of a high number of genomic target sequences. In fact, the present method bypasses the necessity of compatible restriction cleavage sites between two adjacent sequences, thus allowing the selection of a higher number of sequences with respect of those selected according to the prior art, as they are shorter but highly specific.

For example, polyprobes for the simultaneous detection of 6 viruses are already known. The polyprobe according to the present invention, comprises 10 genomic target sequences and is suitable for the simultaneous detection of 10 viruses, therefore improving efficacy of known polyprobes of at least 40%.

Furthermore, polyprobes for the detection of viral infections in globe artichoke are not known at all.

According to the present invention, a kit comprising at least one polyprobe according to the present invention, is useful for the detection of viruses of interest, in an artichoke tissue sample.

A kit according to the present invention is suitable for the simultaneous detection of at least 2 plant viruses of interest in an artichoke tissue sample, particularly suitable for the simultaneous detection of at least 2 viruses in an artichoke tissue sample.

For example, a kit according to the present invention is useful for the simultaneous detection of at least 2 viruses of interest in a artichoke's tissue sample, particularly useful for the simultaneous detection of at least 2 viruses in an artichoke's tissue sample.

A kit according to the present invention, comprises at least one aliquot of at least one polyprobe that is suitable for the simultaneous detection of at least 2 viruses in an artichoke's tissue sample.

Said kit comprises said at least one aliquot of at least one polyprobe that is suitable for the use at a concentration ranging from 1 to 500 ng/ml, preferably ranging from 10 to 400 ng/ml, more preferably ranging from 20 to 300 ng/ml, more preferably ranging from 30 to 250 ng/ml, more preferably ranging from 40 to 200 ng/ml, most preferably ranging from 50 to 150 ng/ml.

The kit comprises at least one nylon membrane, divided into squares, at least comprising one negative control and one positive control. The negative control may be juice extract from a not infected plant, for example a not infected artichoke, and a positive control, i.e. an aliquot of not labeled polyprobe, for example 1ng of not labeled polyprobe.

The negative control may be juice extract from a not infected plant of the same species of the samples to be analyzed.

The samples and the controls may be pretreated with a solution comprising NaOH and EDTA before the application onto the membrane; the membrane may be air dried, preferably at room temperature, for 5-30 min, preferably 15 min, and then exposed to UV rays for 10 sec to 10 min, preferably 5 min.

The membrane loaded with the samples and the controls, after having been air dried and UV exposed, may be stored up to 5 years, preferably at room temperature, for example in a plastic bag, wrapped in standard laboratory filter paper (e.g. Whatman). The hybridization signals may be revealed with suitable detection kits, for example with the DIG luminescent detection kit (Roche, Germany) or by a chromogenic reaction with the DIG Nucleic acid detection kit (Roche, Germany) following the manufacturer's instructions, if the polyprobe has been labeled with digoxigenin, i.e. DIG-UTP.

A kit according to the present invention comprises at least one polyprobe suitable for the simultaneous detection of 10 viruses in artichoke samples.

In a preferred embodiment, a single nylon membrane may be simultaneously loaded with 1 to 100 samples.

Said single nylon membrane may be simultaneously loaded preferably with 20 samples.

A kit according to the present invention is useful for the detection of plant viruses, in plant samples, and particularly useful for the simultaneous detection of at least 2 viruses in plant samples.

Preferably, it is useful for the simultaneous detection of 10 viruses in plant samples, more preferably in artichoke samples.

According to the present invention, one of the major advantages is that several plant samples may be analyzed simultaneously.

According to the present invention, another of the major advantages is that more than a virus species can be detected in the same plant sample, in case of mixed infections. Thus, the kit may be used for simultaneous diagnosis of several harmful viruses affecting the quality of plant production, in particular in nursery vegetable productions, also for phytosanitary certification purposes.

A method for the detection of plant viruses, in plant samples, which contemplates the use of a kit according to the present invention, is therefore also available.

The above mentioned method for the detection of plant viruses at least comprises the steps of
a. selecting an appropriate number of plant samples,
b. treating said samples with a NaOH - EDTA solution,
c. blotting said treated samples onto a nylon membrane,
d. hybridizing said plated samples with a polyprobe according to the present invention,
e. detecting the formed hybrids.

Particularly, it comprises the steps of
a. selecting an appropriate number of artichoke samples,
b. treating said samples with a NaOH - EDTA solution,
c. blotting said treated samples onto a nylon membrane,
d. hybridizing said plated samples with the polyprobe of SEQ. ID NO. 23,
e. detecting the formed hybrids.

More particularly, said formed hybrids are detected by means of a chemiluminescent signal or of a chromogenic signal.

The process for the synthesis of a polyprobe which the present invention relates to, advantageously allows to reduce time of working, save costs and save materials, in particular bypassing all the preliminary operations required to carry out known processes, i.e. obtain plant samples that are infected by the specific virus of interest, extract the totality of the nucleic acids from said samples, isolate the viral sequences of interest and clone said sequences; this advantages are obtained, in particular, by means of synthetic single-stranded oligonucleotides, which the present invention relates to, which make the present process for the synthesis of polybrobes, which the present invention relates to, surprisingly faster, easier and cheaper compared to known processes. In particular, the present process allows the selection and the use of target sequences that are specific, small and, preferably, all of the same length. All these features are very difficult to obtain by means of the known methods due to the absence of suitable restriction sites.

By means of the process which the present invention relates to, it is possible to synthesize polyprobes comprising a surprisingly high number of target sequences of viruses of interest.

Said polyprobes could be advantageously enclosed in commercial kits for the simultaneous detection of several plant viruses; said commercial kits advantageously make the mass analysis of plant samples easier and faster, providing the advantage to simplify and speed up the analysis of nursery vegetable productions and consequently certification procedures.

The present invention could be better explained by the following examples.

It is clear to a person skilled in the art that the following examples are mere representation of the present invention and such examples are not to be considered as a limitation of the scope of the present invention.

### EXAMPLES

### 1. Genomic target sequences

Genomic sequences of 10 viruses affecting artichoke of interest, available in NCBI (http://www.ncbi.nlm.nih.gov) database, *Artichoke Italian latent virus* **AILV** (Acc.n.X87254), *Artichoke mottled crinkle virus* **AMCV** (Acc.n.X51456), *Artichoke latent virus* **ArLV** (Acc.n.X87255), *Bean yellow mosaic virus* **BYMV** (Acc.n.AB041972) *Cucumber mosaic virus* **CMV** (Acc.n.D00355), *Pelargonium zonate spot virus* **PZSV** (Acc.n.AJ272329), *Tomato infectious chlorosis virus* **TICV** (Acc.n.NC013259), *Tobacco mosaic virus* **TMV** (Acc.n. AB369276.1), *Tomato spotted wilt virus* **TSWV** (Acc.n.AB010996), *Turnip mosaic virus* **TuMV** (Acc.n.AF071526), have been analyzed by means of the software BLAST (Basic Alignment Search Tool http://www.blast.ncbi.nlm.nih.gov), in order to select genomic target sequences, each one having a length of 50nt, highly specific for the detection of the correspondent virus of interest.

The selected genomic target sequences are the following:
**AILV**
**AMCV**
**ArLV**
**BYMV**
**CMV**
**PZSV**
**TICV**
**TMV**
**TSWV** **TuMV**

### 2. Synthesis of 4 single-stranded DNA (ssDNA) oligonucleotides: ssAiBy, ssArAm, ssCmTmTi, ssTsPzTu

The above selected genomic target sequences, each one having a length of 50nt, have been joined in tandem in order to obtain 4 single-stranded DNA (ssDNA) oligonucleotides, each one containing 2 or 3 selected sequences joined together, called respectively ssAiBy, ssArAm, ssCmTmTi, ssTsPzTu. The synthesis of the single-stranded DNA oligonucleotides has been commissioned to an external service (Primm s.r.l., Milan).

In detail, the selected genomic target sequences have been combined as follows:
- AILV (SEQ. ID NO. 1) and BYMV (SEQ. ID. NO. 4) in order to obtain the single-stranded DNA oligonucleotide **ssAiBy** having the following sequence:
- ArLV (SEQ. ID NO. 3) and AMCV (SEQ. ID. NO. 2) in order to obtain the single-stranded DNA oligonucleotide **ssArAm** having the following sequence:
- CMV (SEQ. ID NO. 5), TMV (SEQ. ID. NO. 8) and TICV (SEQ. ID NO. 7) in order to obtain the single-stranded DNA oligonucleotide **ssCmTmTi** having the following sequence:
- TSWV (SEQ. ID NO. 9), PZSV (SEQ. ID. NO. 6) and **TuMV** (SEQ. ID NO. 10) in order to obtain the single-stranded DNA oligonucleotide **ssTsPzTu** having the following sequence:

Said 4 single-stranded DNA oligonucleotides, namely **ssAiBy, ssArAm, ssCmTmTi** and **ssTsPzTu**, have been employed in PCR reactions with specific primers (forward and reverse) in order to synthetize the correspondent double-stranded DNA (dsDNA) oligonucleotides, i.e. the genic targets of interest, namely **dsAiBy, dsArAm, dsCmTmTi** and **dsTsPzTu.**

The operative conditions were as follows:
• Synthesis of **dsAiBy:**

| | | |
|---|---|---|
| Primer forward | 5'ATTCACTAGTCCCTATTTAG3' | (SEQ. ID NO. 15) |
| Primer reverse | 5'AGATCAAGCTCACACGAGG3' | (SEQ. ID NO. 16) |

Amplification program: 94°C for 2 min, (94°C for 30 sec, 50°C for 30 sec, 72°C for 15 sec) for 35 cycles, 72°C for 5 min
• Synthesis of **dsArAm:**

| | | |
|---|---|---|
| Primer forward | 5'TTGTTCATAAGGGAGCGCGT3' | (SEQ. ID NO. 17) |
| Primer reverse | 5'CCGAATCTTTATCAAAGTACATAGCC3' | (SEQ. ID NO. 18) |

Amplification program: 94°C for 2 min, (94°C for 30 sec, 54°C for 30 sec, 72°C for 15 sec) for 35 cycles, 72°C for 5 min
• Synthesis of **dsCmTmTi:**

| | | |
|---|---|---|
| Primer forward | 5'GTTTATTTACAAGAGCGTACGG3' | (SEQ. ID NO. 19) |
| Primer reverse | 5'CACAGTATACAGCAGCGGCAG3' | (SEQ. ID NO. 20) |

Amplification program: 94°C for 2 min, (94°C for 30 sec, 55°C for 30 sec, 72°C for 15 sec) for 35 cycles, 72°C for 5 min
• Synthesis of **dsTsPzPu:**

| | | |
|---|---|---|
| Primer forward | 5'GANGAACATCTTCCTTTGG3' | (SEQ. ID NO. 21), |

where"N" could be "A" or "G"

| | | |
|---|---|---|
| Primer reverse | 5'GCGCCACGCAGTGCTG3' | (SEQ. ID NO. 22) |

Amplification program: 94°C for 2 min, (94°C for 30 sec, 54°C for 30 sec, 72°C for 15 sec) for 35 cycles, 72°C for 5 min The synthesis of the dsDNA oligonucleotides has been carried out by means of end-point PCR

### 3. Synthesis of the recombinant plasmids pAiBy, pArAm, pCmTmTi and pTsPzTu.

The cloning in suitable vectors of said dsDNA oligonucleotides has been carried out following standard methods (Sambrook et al.,1989, Molecular Cloning: A Laboratory Manual. New York: Cold Spring Harbor Laboratory), as follows. Said 4 double-stranded DNA oligonucleotides, namely **dsAiBy, dsArAm, dsCmTmTi** and **dsTsPzTu**, have been employed to carry out 4 independent ligation reactions in presence of the cloning vector pGem-T Easy (Promega) in order to produce 4 recombinant plasmids, namely **pAiBy**, **pArAm**, **pCmTmTi** and **pTsPzTu,** each plasmid containing the correspondent double-stranded DNA oligonucleotide. The ligation of said 4 double-stranded DNA oligonucleotides into said 4 plasmids has been carried out by means of the protruding T (thymine) residues of the commercial plasmid, following the instructions of the manufacturer.

### 4. Synthesis of the recombinant plasmids pArAmTsPzTu and pAiByCmTmTi

Recombinant plasmids pArAm and pTsPzTu have been enzymatically digested by means of restriction enzyme *Eco*RI*.* The products of the digestion reaction, having compatible *Eco*RI 5' protruding terminals, have been ligated into a single cloning vector, i.e. pGem-T Easy (Promega), in order to obtain the recombinant plasmid **pArAmTsPzTu,** containing the double-stranded DNA oligonucleotide **dsArAmTsPzTu.** Said cloning vector pGem-T Easy (Promega) was previously linearized by means of *Eco*RI and dephosphorylated.

With a similar procedure, recombinant plasmids pAiBy and pCmTmTi have been enzymatically digested by means of restriction enzyme *Eco*RI*.* The products of the digestion reaction, having compatible *Eco*RI 5' protruding terminals, have been ligated.

The product of the ligation reaction has been amplified by means of PCR under the following conditions:

| | | |
|---|---|---|
| Primer forward | 5'ATTCACTAGTCCCTATTTAG3' | (SEQ. ID NO. 15) |
| Primer reverse | 5'CACAGTATACAGCAGCGGCAG3' | (SEQ. ID NO. 20) |

Amplification program: 94°C for 3 min, (94°C for 30 sec, 49°C for 30 sec, 72°C for 30 sec) for 35 cycles, 72°C for 3 min
in order to synthesize the double-stranded DNA oligonucleotide **dsAiByCmTmTi** employed in the ligation reaction with the plasmid pGem-T Easy (Promega), in order to obtain the recombinant plasmid **pAiByCmTmTi,** containing the double-stranded DNA oligonucleotide dsAiByCmTmTi. Said cloning vector pGem-T Easy (Promega) was previously linearized by means of *Eco*RI and dephosphorylated.

### 5. Synthesis of the recombinant plasmid pAiByCmTmTiTuPzTsArAm

Recombinant plasmids pArAmTsPzTu and pAiByCmTmTi have been subjected to a double enzymatic digestion with enzymes *Nco*I and *Pst*I, and the two products of the digestion reaction, having compatible terminals, namely the fragments CmTmTi and ArAmTsPzTu, have been ligated.

The product of the ligation reaction has been amplified by means of PCR under the following conditions:

| | | |
|---|---|---|
| Primer forward | 5'GTTTATTTACAAGAGCGTACGG3' | (SEQ. ID NO. 19) |
| Primer reverse | 5'CCGAATCTTTATCAAAGTACATAGCC3' | (SEQ. ID NO. 18) |

Amplification program: 94°C for 3 min (94°C for 30 sec, 52°C for 30 sec, 72°C for 1 min) for 35 cycles, 72°C for 5 min.

The product of the amplification reaction has been inserted into the cloning vector pGem-T Easy (Promega), obtaining the recombinant plasmid **pCmTmTiArAmTsPzTu.**

Recombinant plasmids pCmTmTiArAmTsPzTu and pAiBy have been enzymatically digested by means of restriction enzyme *Not*I*.* The products of the digestion reaction, having compatible terminals, have been ligated.

The product of the ligation reaction has been amplified by means of PCR under the following conditions:

| | | |
|---|---|---|
| Primer forward | 5'GTTTATTTACAAGAGCGTACGG3' | (SEQ. ID NO. 19) |
| Primer reverse | 5'AGATCAAGCTCACACGAGG3' | (SEQ. ID NO. 16) |

Amplification program: 94°C for 2 min, (94°C for 30 sec, 55°C for 30 sec, 72°C for 40 sec) for 35 cycles, 72°C for 5 min,
and then inserted into the cloning vector pGem-T Easy (Promega), obtaining the recombinant plasmid **pAiByCmTmTiTuPzTsArAm,** containing the full length sequence of the polyprobe.

The 569 bp sequence of the polyprobe is the following:

### 6. Synthesis of the polyprobe for the simultaneous detection of 10 viruses in artichoke

The polyprobe has been synthesized by means of end-point PCR carried out employing the plasmid pAiByCmTmTiTuPzTsArAm, which contains the multiple recombinant sequence of 10 specific portions of viral genomes, i.e. the entire sequence of the polyprobe.

The synthesis of the polyprobe has been carried out by means of PCR DIG probe synthesis kit (Roche), with Digoxigenin-11-UTP (DIG-UTP) as marker molecule.

In detail, 40ng of plasmid pAiByCmTmTiTuPzTsArAm have been applied onto Whatman FTA card (Whatman FTA kit pK/1, GE healthcare life Sciences, Germany) disks, following the manufacturer's instructions, and used as template for a PCR reaction in the following conditions:

| | | |
|---|---|---|
| Primer forward | 5'ATTCACTAGTCCCTATTTAG3' | (SEQ. ID NO. 15) |
| Primer reverse | 5'CCGAATCTTTATCAAAGTACATAGCC3' | (SEQ. ID NO. 18) |

Amplification program: 94°C for 2 min, (94°C for 50 sec, 50°C for 30 sec, 72°C for 40 sec) for 35 cycles, 72°C for 5 min.

The 569 bp sequence of the polyprobe is the following: The polyprobe (SEQ. ID NO. 23) is therefore labeled with DIG-UTP.

After the synthesis, the polyprobe is stored at -20°C.

For the use in the diagnostic kit of the present invention the DIG-UTP labeled polyprobe could be used at a concentration of 50-150 ng of polyprobe/ml of hybridization solution. **7. Preparation and validation of a commercial diagnostic kit for the detection of**

### 10 viruses in artichoke

Aliquots of the polyprobe are enclosed in a commercial diagnostic kit. Each aliquot is sufficient for the hybridization of a membrane in a range from 50ng of polyprobe/ml of hybridization solution, to 150ng of polyprobe/ml of hybridization solution.

Said commercial diagnostic kit is useful to detect 10 phytoviruses in artichoke samples.

The method of detection is based on the molecular hybridization of the labeled DNA polyprobe with specific genomic viral sequences.

The hybridization occurs on a nylon membrane, and the signal is obtained by means of a Fragment antigen-binding (Fab), that binds digoxigenin, conjugated to alkaline phosphatase.

The anti-digoxigenin Fab fragments conjugated to the alkaline phosphatase and its CDP-star substrate are both produced by Roche; the reaction of the alkaline phosphatase with its substrate CDP-star produces a chemiluminescent signal, which could be detected following the manufacturer's instructions.

The nylon membrane of the commercial diagnostic kit, previously divided into squares of 1 cm2, contains: a negative control, namely juice extracted from a healthy artichoke, and a positive control, i.e. 1 ng of not labeled polyprobe, said not labeled polyprobe has been synthesized by means of Whatman FTA card (Whatman FTA kit pK/1, GE healthcare life Sciences, Germany) as previously described (Minutillo et al. 2012 EUROPEAN JOURNAL OF PLANT PATHOLOGY, vol. 134, p. 459-46).

Before being applied onto the membrane, the negative control, the positive control and all the samples to be assayed are treated with a freshly prepared NaOH 50 mM - EDTA 2.5 mM solution with a 1:6 ratio (w/v).

Once controls and samples have been loaded on the membrane, the membrane is air-dried at room temperature for 15 min and then exposed to UV rays for 5 minutes. Once the membrane has been exposed to UV rays, it could be used immediately or it could be stored at room temperature between 2 Whatman paper sheets (3MM).

For the hybridization, the membrane containing controls and samples is incubated at 40°C overnight in 150 µl/cm2 of DIG Easy Hyb solution (Roche, Germany) and the polyprobe at a concentration of 135 ng/ml of hybridization solution.

After the hybridization, the membrane is washed four times with SSC 2x-SDS 0,1% buffer, at 65°C, for 15 min each time; then the membrane is washed once at room temperature, for 5 min with the Washing Buffer of the DIG luminescent detection kit (Roche, Germany), following the manufacturer's instructions.

The chemiluminescent signal produced by the formed hybrids has been detected by means of CDP-star (Roche, Germany), following the manufacturer's instructions.

### 8. Results of the tests

Under the above described conditions, the polyprobe has been able to produce hybridization signals in presence of all the positive controls, while no hybridization signals have been produced in presence of the negative control, i.e. juice from healthy artichoke.

## Claims

1. A method of synthesis of a polyprobe, comprising at least the following phases:
a) selecting 10 genomic target sequences, of 10 globe artichoke's viruses, one sequence for each one of said 10 viruses,
b) synthesizing 4 single-stranded oligonucleotides comprising at least 2 of said genomic target sequences joined together,
c) synthesizing 4 double-stranded oligonucleotides corresponding to said 4 single-stranded oligonucleotides,
d) cloning said 4 double-stranded oligonucleotides into 4 suitable vectors, which are subcloned in tandem obtaining a recombinant cloning vector comprising the entire sequence of said polyprobe,
e) labeling said polyprobe.

2. A method according to claim 1, **characterized in that** said genomic target sequences are all of the same length.

3. A method according to claim 1, **characterized in that** said viruses are selected from: *Artichoke Italian latent virus* (AILV); *Artichoke latent virus* (ArLV); *Artichoke mottled crinkle virus* (AMCV); *Turnip mosaic virus* (TuMV); *Tomato infectious chlorosis virus* (TICV); *Bean yellow mosaic virus* (BYMV); *Cucumber mosaic virus* (CMV); *Pelargonium zonate spot virus* (PZSV); *Tomato spotted wilt virus* (TSWV), *Tobacco mosaic virus* (TMV), *Artichoke Aegean ringspot virus* (AARSV), *Artichoke yellow ringspot virus* (AYRSV), *Tomato black ring virus* (TBRV), *Artichoke vein banding virus* (AVBV), *Broad bean wilt virus* (BBWV), *Tobacco streak virus* (TSV), *Artichoke latent virus M* (ArLVM), *Artichoke latent virus S* (ArLVS), *Potato virus X* (PVX), *Artichoke curly dwarf virus* (ACDV), *Tobacco rattle virus* (TRV).

4. A method according to claim 1, **characterized in that** said 4 single-stranded oligonucleotides have the following sequences: and

5. A nucleic acid polyprobe for the simultaneous detection of at least two plant viruses, based on specific genomic target sequences of plant viruses affecting an artichoke, **characterized in that** it comprises 10 genomic target sequences of plant viruses, each one from a different virus species, and **in that** it has the following sequence:

6. A nucleic acid polyprobe according to claim 5 **characterized in that** is a DNA polyprobe.

7. A kit comprising at least one polyprobe according to claim 5, for the detection of viruses, in an artichoke tissue sample.

8. Use of a kit according to claim 7 for the simultaneous detection of at least 2 viruses in an artichoke's tissue sample.

9. Use of a kit according to claim 8 for the simultaneous detection 10 viruses in an artichoke's tissue sample.

## Patentansprüche

1. Verfahren zur Synthese einer Polyprobe, umfassend mindestens die folgenden Phasen:
a) Auswählen von 10 genomischen Zielsequenzen von 10 Artischockenviren, eine Sequenz für jedes der 10 Viren,
b) Synthetisieren von 4 einzelsträngigen Oligonukleotiden, umfassend mindestens 2 der genannten genomischen Zielsequenzen miteinander verbunden,
c) Synthetisieren von 4 doppelsträngigen Oligonukleotiden, die den 4 einzelsträngigen Oligonukleotiden entsprechen,
d) Klonieren der 4 doppelsträngigen Oligonukleotiden in 4 geeignete Vektoren, die in tandem subkloniert werden, wobei ein rekombinanter Klonierungsvektor erhalten wird, der die gesamte Sequenz der Polyprobe umfasst,
e) Markieren der Polyprobe.

2. Verfahren nach Anspruch 1, **dadurch gekennzeichnet, dass** die genomischen Zielsequenzen alle die gleiche Länge haben.

3. Verfahren nach Anspruch 1, **dadurch gekennzeichnet, dass** die Viren ausgewählt sind aus: *Artichoke Italian latent virus* (AILV), *latentes Artichoken-Virus* (ArLV); *Artichoke mottled crinkle virus* (AMCV); *Turnip mosaic virus* (TuMV); *Tomato infectious chlorosis virus* (TICV); *Bean yellow mosaic virus* (BYMV); *Gurkenmosaikvirus* (CMV); *Pelargonium zonate spot virus* (PZSV); *Tomatenbronzefleckenvirus* (TSWV), *Tabakmosaikvirus* (TMV), *Artichoke Aegean ringspot virus* (AARSV), *Artichoke yellow ringspot virus* (AYRSV), *Tomato black ring virus* (TBRV), *Artichoke vein banding virus* (AVBV), *Broad bean wilt virus* (BBWV), *Tobacco streak virus* (TSV), *Artichoke latent virus M* (ArLVM), *Artichoke latent virus S* (ArLVS), *Kartoffelvirus X* (PVX), *Artichoke curly dwarf virus* (ACDV), *Tabak-Rattle-Virus* (TRV).

4. Verfahren nach Anspruch 1, **dadurch gekennzeichnet, dass** die 4 einzelsträngigen Oligonukleotide die folgenden Sequenzen aufweisen: and

5. Nukleinsäure-Polyprobe zum simultanen Nachweis von mindestens zwei Pflanzenviren, basierend auf spezifischen genomischen Zielsequenzen von Pflanzenviren, die eine Artischocke betreffen könnten, **dadurch gekennzeichnet, dass** sie 10 genomische Zielsequenzen von Pflanzenviren umfasst, jeweils von einer anderen Virusspezies, und wobei sie die folgende Sequenz umfasst:

6. Nukleinsäure-Polyprobe nach Anspruch 5, **dadurch gekennzeichnet, dass** es sich um eine DNA-Polyprobe handelt.

7. Kit, umfassend mindestens eine Polyprobe nach Anspruch 5 zum Nachweis von Viren in einer Artischocken-Gewebeprobe.

8. Verwendung eines Kits nach Anspruch 7 zum gleichzeitigen Nachweis von mindestens 2 Viren in einer Artischocken-Gewebeprobe.

9. Verwendung eines Kits nach Anspruch 8 zum gleichzeitigen Nachweis von 10 Viren in einer Artischocke-Gewebeprobe.

## Revendications

1. Procédé de synthèse d'une polysonde, comprenant au moins les phases suivantes :
a) la sélection de 10 séquences génomiques cibles, de 10 virus d'artichaut, une séquence pour chacun desdits 10 virus,
b) la synthèse de 4 oligonucléotides à simple brin comprenant au moins 2 desdites séquences génomiques cibles liées ensemble,
c) la synthèse de 4 oligonucléotides à double brin correspondant auxdits 4 oligonucléotides à simple brin,
d) le clonage desdits 4 oligonucléotides à double brin dans 4 vecteurs appropriés, qui sont sous-clonés en tandem pour obtenir un vecteur de clonage recombinant comprenant la séquence entière de ladite polysonde,
e) le marquage de ladite polysonde.

2. Procédé selon la revendication 1, **caractérisé en ce que** lesdites séquences génomiques cibles ont toutes la même longueur.

3. Procédé selon la revendication 1, **caractérisé en ce que** lesdits virus sont sélectionnés parmi : le virus latent italien de l'artichaut (AILV) ; le virus latent de l'artichaut (ArLV) ; le virus de la frisolée marbrée de l'artichaut (AMCV) ; le virus de la mosaïque du navet (TuMV) ; le virus de la chlorose infectieuse de la tomate (TICV) ; le virus de la mosaïque jaune du haricot (BYMV) ; le virus de la mosaïque du concombre (CMV) ; le virus des bandes concentriques du pélargonium (PZSV) ; le virus de la maladie bronzée de la tomate (TSWV), le virus de la mosaïque du tabac (TMV), le virus des taches en anneaux de l'artichaut égéen (AARSV), le virus des taches jaunes en anneaux de l'artichaut (AYRSV), le virus des anneaux noirs de la tomate (TBRV), le virus du liséré des nervures de l'artichaut (AVBV), le virus du flétrissement de la fève (BBWV), le virus des stries du tabac (TSV), le virus latent M de l'artichaut (ArLVM), le virus latent S de l'artichaut (ArLVS), le virus X de la pomme de terre (PVX), le virus du nanisme enroulant de l'artichaut (ACDV), le virus du bruissement du tabac (TRV).

4. Procédé selon la revendication 1, **caractérisé en ce que** lesdits 4 oligonucléotides à simple brin possèdent les séquences suivantes : et

5. Polysonde d'acide nucléique pour la détection simultanée d'au moins deux virus de plante, sur la base de séquences génomiques cibles spécifiques de virus de plante affectant un artichaut, **caractérisée en ce qu'**elle comprend 10 séquences génomiques cibles de virus de plante, chacune provenant d'une espèce différente de virus, et **en ce qu'**elle possède la séquence suivante :

6. Polysonde d'acide nucléique selon la revendication 5 **caractérisée en ce que** c'est une polysonde d'ADN.

7. Kit comprenant au moins une polysonde selon la revendication 5, pour la détection de virus, dans un échantillon de tissu d'artichaut.

8. Utilisation d'un kit selon la revendication 7 pour la détection simultanée d'au moins 2 virus dans un échantillon de tissu d'artichaut.

9. Utilisation d'un kit selon la revendication 8 pour la détection simultanée de 10 virus dans un échantillon de tissu d'artichaut.


### SEQUENCE LISTING

<110> Agritest s.r.l.
<120> Synthesis and use of a polyprobe for the detection of viral infections in globe artichoke
<130> 14132AA37
<160> 23
<170> PatentIn version 3.5
<210> 1
   <211> 50
   <212> DNA
   <213> Artichoke italian latent virus
<400> 1
   attcactagt ccctatttag gaatggccat ctggtatgtt tttgatgctt 50
<210> 2
   <211> 50
   <212> DNA
   <213> Artichoke mottled crinkle virus
<400> 2
   cgcaaccact gaggtggggc gagtggctat gtactttgat aaagattcgg 50
<210> 3
   <211> 50
   <212> DNA
   <213> Artichoke latent virus
<400> 3
   ttgttcataa gggagcgcgt taaggcctgg gaatttgtac ccgagatgca 50
<210> 4
   <211> 50
   <212> DNA
   <213> bean yellow mosaic virus
<400> 4
   actctgacag ggtaagcagt tagtgaggtt gcctcgtgtg agcctgatct 50
<210> 5
   <211> 50
   <212> DNA
   <213> cucumber mosaic virus
<400> 5
   gtttatttac aagagcgtac ggttcaaccc ctgcctcccc tgtaaaactc 50
<210> 6
   <211> 50
   <212> DNA
   <213> Pelargonium zonate spot virus
<400> 6
   atgcccccta agagacagaa cactgagacg cgcaaagcta gacaaaaccg 50
<210> 7
   <211> 50
   <212> DNA
   <213> Tomato infectious chlorosis virus
<400> 7
   tccatgcagg aggataataa acgaaccatc tgccgctgct gtatactgtg 50
<210> 8
   <211> 50
   <212> DNA
   <213> Tobacco mosaic virus
<400> 8
   cttacagtat cactactcca tctcagttcg tgttcttgtc atcagcgtgg 50
<210> 9
   <211> 50
   <212> DNA
   <213> Tomato spotted wilt virus
<400> 9
   gaaggaacat cttcctttgg aacctatgag tctgattcta tcacagaatc 50
<210> 10
   <211> 50
   <212> DNA
   <213> Turnip mosaic virus
<400> 10
   cgtgcgagag aagcacacat ccagatgaaa gcagcagcac tgcgtggcgc 50
<210> 11
   <211> 100
   <212> DNA
   <213> Artificial Sequence
<220>
   <223> single-stranded DNA oligonucleotide
<400> 11
   attcactagt ccctatttag gaatggccat ctggtatgtt tttgatgctt actctgacag 60
   ggtaagcagt tagtgaggtt gcctcgtgtg agcctgatct 100
<210> 12
   <211> 100
   <212> DNA
   <213> Artificial Sequence
<220>
   <223> single-stranded DNA oligonucleotide
<400> 12
   ttgttcataa gggagcgcgt taaggcctgg gaatttgtac ccgagatgca cgcaaccact 60
   gaggtggggc gagtggctat gtactttgat aaagattcgg 100
<210> 13
   <211> 150
   <212> DNA
   <213> Artificial Sequence
<220>
   <223> single-stranded DNA oligonucleotide
<400> 13
   gtttatttac aagagcgtac ggttcaaccc ctgcctcccc tgtaaaactc cttacagtat 60
   cactactcca tctcagttcg tgttcttgtc atcagcgtgg tccatgcagg aggataataa 120
   acgaaccatc tgccgctgct gtatactgtg 150
<210> 14
   <211> 150
   <212> DNA
   <213> Artificial Sequence
<220>
   <223> single-stranded DNA oligonucleotide
<400> 14
   gaaggaacat cttcctttgg aacctatgag tctgattcta tcacagaatc atgcccccta 60
   agagacagaa cactgagacg cgcaaagcta gacaaaaccg cgtgcgagag aagcacacat 120
   ccagatgaaa gcagcagcac tgcgtggcgc 150
<210> 15
   <211> 20
   <212> DNA
   <213> Artificial Sequence
<220>
   <223> PCR primer forward
<400> 15
   attcactagt ccctatttag 20
<210> 16
   <211> 19
   <212> DNA
   <213> Artificial Sequence
<220>
   <223> PCR primer reverse
<400> 16
   agatcaagct cacacgagg 19
<210> 17
   <211> 20
   <212> DNA
   <213> Artificial Sequence
<220>
   <223> PCR primer forward
<400> 17
   ttgttcataa gggagcgcgt 20
<210> 18
   <211> 26
   <212> DNA
   <213> Artificial Sequence
<220>
   <223> PCR primer reverse
<400> 18
   ccgaatcttt atcaaagtac atagcc 26
<210> 19
   <211> 22
   <212> DNA
   <213> Artificial Sequence
<220>
   <223> PCR primer forward
<400> 19
   gtttatttac aagagcgtac gg 22
<210> 20
   <211> 21
   <212> DNA
   <213> Artificial Sequence
<220>
   <223> PCR primer reverse
<400> 20
   cacagtatac agcagcggca g 21
<210> 21
   <211> 19
   <212> DNA
   <213> Artificial Sequence
<220>
   <223> PCR primer forward
<220>
   <221> misc_feature
   <222> (3)..(3)
   <223> "n" could be "a" or "g"
<400> 21
   gangaacatc ttcctttgg 19
<210> 22
   <211> 16
   <212> DNA
   <213> Artificial Sequence
<220>
   <223> PCR primer reverse
<400> 22
   gcgccacgca gtgctg 16
<210> 23
   <211> 569
   <212> DNA
   <213> Artificial Sequence
<220>
   <223> full length sequence of the polyprobe
<400> 23
   attcactagt ccctatttag gaatggccat ttggtatgtt tttgatgctt actctgacag      60
   ggtaagcagt tagtgaggtt gcctcgtgtg agcctgatct aatcactagt gaattcgcgg     120
   ccgcgggaat tcgattgttt atttacaaga gcgtacggtt caacccctgc ctcccctgta     180
   aaactcctta cagtatcact actccatctc agttcgtgtt cttgtcatca gcgggtccat     240
   gcaggaggat aataaacgaa ccatctgccg ctgctgtata ctgtgaatca ctagtgaatt     300
   cgccacgcag tgctgctgct ttcatctgga tgtgtgcttc tctcgccacg cggttttggt     360
   ctagctttgc gcgtctcagt gttctgtctc ttagggggca tgattctgtg atagaatcag     420
   actcatagtt ccaaaggaag atgtttcctc aatcgaattc actagtgatt ttgttcataa     480
   gggagcgcgg ttaaggctgg gaattgtacc cgagatgcac gcaaccactg aggtggggcg     540
   agtggctatg tactttgata aagattcgg                                       569